(19) Europäisches Patentamt
European Patent Office
Office européen des brevets

(11) Publication number: 0 285 884
A2

# (12) EUROPEAN PATENT APPLICATION

(21) Application number: 88104384.8

(22) Date of filing: 18.03.88

(51) Int. Cl.[4] C07D 405/04 , A61K 31/52 , //C07D307/20

(30) Priority: 20.03.87 US 28817

(43) Date of publication of application:
12.10.88 Bulletin 88/41

(84) Designated Contracting States:
AT BE CH DE ES FR GB GR IT LI LU NL SE

(71) Applicant: Bristol-Myers Company
345 Park Avenue
New York New York 10154(US)

(72) Inventor: Farina, Vittorio
106 Knollwood Road
West Hartford, CT 06110(US)
Inventor: Martin, John C.
40 Brookside Place
Cheshire, CT 06410(US)
Inventor: Benigni, Daniel A-
120 Maccollam Road
Elbridge, NY 13060(US)

(74) Representative: Kinzebach, Werner, Dr.
Patentanwälte Reitstötter, Kinzebach und Partner Sternwartstrasse 4 Postfach 86 06 49
D-8000 München 86(DE)

## (54) Process to produce 2', 3'-Dideoxynucleosides.

(57) There is disclosed a novel process to produce 2′,3′-dideoxynucleosides such as, for example, 2′,3′-dideoxycytidine, in high yields. More particularly, the various stereoisomers of 2′,3′-dideoxynucleosides are obtained. The α-and β-(L)-2′,3′-dideoxynucleosides and certain α-(D)-2′,3′-dideoxynucleosides are obtained as stereochemically pure compounds not heretofore obtained. The compounds so produced are useful as antiviral and antibiotic agents.

EP 0 285 884 A2

# PROCESS TO PRODUCE 2', 3'-Dideoxynucleosides

## BACKGROUND OF THE INVENTION

### Field of the Invention

This invention relates to an improved process to produce 2',3'-dideoxynuclesides. Further, this invention relates to novel α-(D)-and α-and β-(L)-2',3'-dideoxynucleosides.

### Background - Related References

Typically, 2',3'-dideoxycytidine (ddC) is synthesized from 2'-deoxycytidine[1,2]. This is a general method for the synthesis of 2',3'-dideoxynucleosides. The starting materials for this synthesis are, however, extremely expensive and not available in bulk. The reagents required for this deoxygenation, furthermore, are also quite costly.

Other preparations involve the use of cytidine or protected cytidine, which is then subjected to stepwise di-deoxygenation. The original synthesis of Horwitz[3] used a cyclonucleoside as intermediate and involved many steps.

The racemic mixture of β-(D,L)-2',3'-dideoxyadenosine (ddA) has been described in the literature by M.J. Robbins, et al.[4]

Most of the other procedures involve reduction of the nucleoside to a 2',3'-unsaturated intermediate, which is then catalytically hydrogenated [5,6,7,8,9,10]. A more complex approach that involves a Perkow reaction has also been described[11]. A number of these methods are summarized in a U.S. Patent[12]. More recently, a photoreductive synthesis of dideoxynucleosides, also from nucleosides, has appeared[13]. Some of the above methods apply to 2',3'-dideoxyuracil, but this is easily converted into ddC[14].

Finally, a synthesis of dideoxynucleosides,. which are racemic and also anomeric, from a variety of tetrahydrofurans, via a photochemical chlorination, is described in the Russian literature[15]. Cytidine is an expensive starting material for multi-kilo preparations of ddC. In addition, the substituted tetrahydrofurans used by the Russian authors, although rather inexpensive, are racemic and are also anomeric mixtures and would produce ddC as a mixture of difficulty separable enantiomers. Also, L-dideoxynucleosides are unavailable from natural nucleosides. This is the only method known to make them.

## SUMMARY OF THE INVENTION

In summary, this invention comprises a process for producing 2',3'-dideoxynucleosides represented by the formula

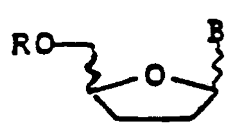

wherein B is a purine or pyrimidine base and R is H or a hydroxy-protecting group, novel 2',3'-dideoxynucleosides thereby produced, and their use as antiviral and antibiotic agents, especially as antiviral agents effective against the acquired immune deficiency syndrome (AIDS) infectious agents.

The earlier application, U.S. Serial No. 028,817 filed 20 March 1987 discloses broadly the process defined herein as the invention. More particularly, the description of the invention and original SCHEMES I and II set forth structural formulas which clearly indicate that product produced according to the process of the invention may possess the base in the β-position or the α-position. But, those formulas less explicitly define the stereochemical orientation with respect to the hydroxymethyl moiety pendant to the pentofuranose ring at the 4-position. The original actual examples illustrate 2',3'-dideoxynucleosides produced

according to the invention starting from (D)-γ-carboxy-γ-butyrolactone. We have now illustrated the process starting from (L)-γ-carboxy-γ-butyrolactone to produce each of the possible enantiomers separately. The process according to this invention comprises a chiral synthesis of each enantiomer and avoids the additional step of separating enantiomers.

In another aspect, this invention comprises (L)-dideoxynucleosides. Particularly, this invention comprises (L)-dideoxynucleosides produced by to the process according to this invention.

In still another aspect, this invention comprises certain intermediates useful in the above-described process of the formulas

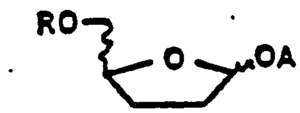

and

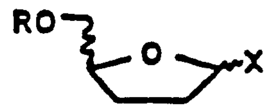

wherein A is an O-activating group as defined below and X is a leaving group selected from F, Cl, Br and I, preferably from Cl and Br.

## DETAILED DESCRIPTION OF THE INVENTION

The present invention is a process for producing a dideoxynucleoside represented by the formula

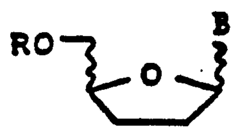

wherein B is a base selected from the group of purine and pyrimidine bases and R is a member selected from H and hydroxy-protecting groups, which comprises the steps of:

(a) converting a member of the group consisting of L-and D-γ-carboxy-γ-butyrolactone of the formula

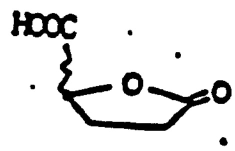

Formula 1

to a 5-O-hydroxy-protecting group-γ-butyrolactone of the formula

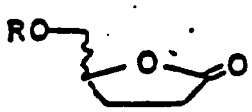

Formula 2

wherein R is a hydroxy-protecting group;

(b) converting the intermediate from step (a), Formula 2, to the 5-O-hydroxy-protecting group-methyl-2,3-dideoxy-D-or -L-pentofuranose of the formula

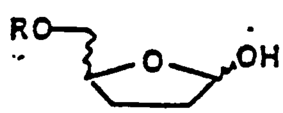

Formula 3

wherein R is a hydroxy-protecting group;

(c) converting the intermediate from step (b), Formula 3, to the 1-O-activating-group-5-O-hydroxy-protecting group-2,3-dideoxy-D-or -L-pentofuranose of the formula

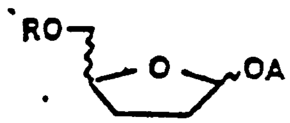

Formula 4

wherein R is a hydroxy-protecting group and A is an O-activating group selected from alkylcarbonyl, arylcarbonyl, alkylthiocarbonyl, arylthiocarbonyl, alkylsulfonyl, arylsulfonyl and carbonate groups wherein the alkyl moiety may be an unsubstituted or substituted $C_1$-$C_3$ alkyl group and the aryl moiety may be an unsubstituted or substituted phenyl group and wherein the substituent on the alkyl and aryl moieties may be selected from 1 to 3 groups selected from halo and $C_1$-$C_3$ alkoxy groups;

(d) converting the intermediate from step (c), Formula 4, by reaction with a compound having one of the formulas HX and TMSX to the 1-leaving-group-5-O-protecting group-2,3-dideoxy-D-or -L-pentofuranose of the formula

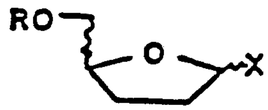

Formula 5

wherein R is a hydroxy-protecting group and X is a leaving group selected from F, Cl, Br and I;

(e) reacting the intermediate from step (d), Formula 5, with an activated base selected from purine and pyrimidine bases, wherein the base has been activated by means of reacting the pendant amino and hydroxy groups on the nucleus of the given base with an activating compound selected from silylating and acetylating and benzoylating agents, in the presence of one of a Bronsted acid and a Lewis acid; and

(f) recovering the 2',3'-dideoxynucleoside from step (e) above.

The process for producing two representative dideoxynucleosides according to this invention is outlined in Scheme I below.

Scheme I

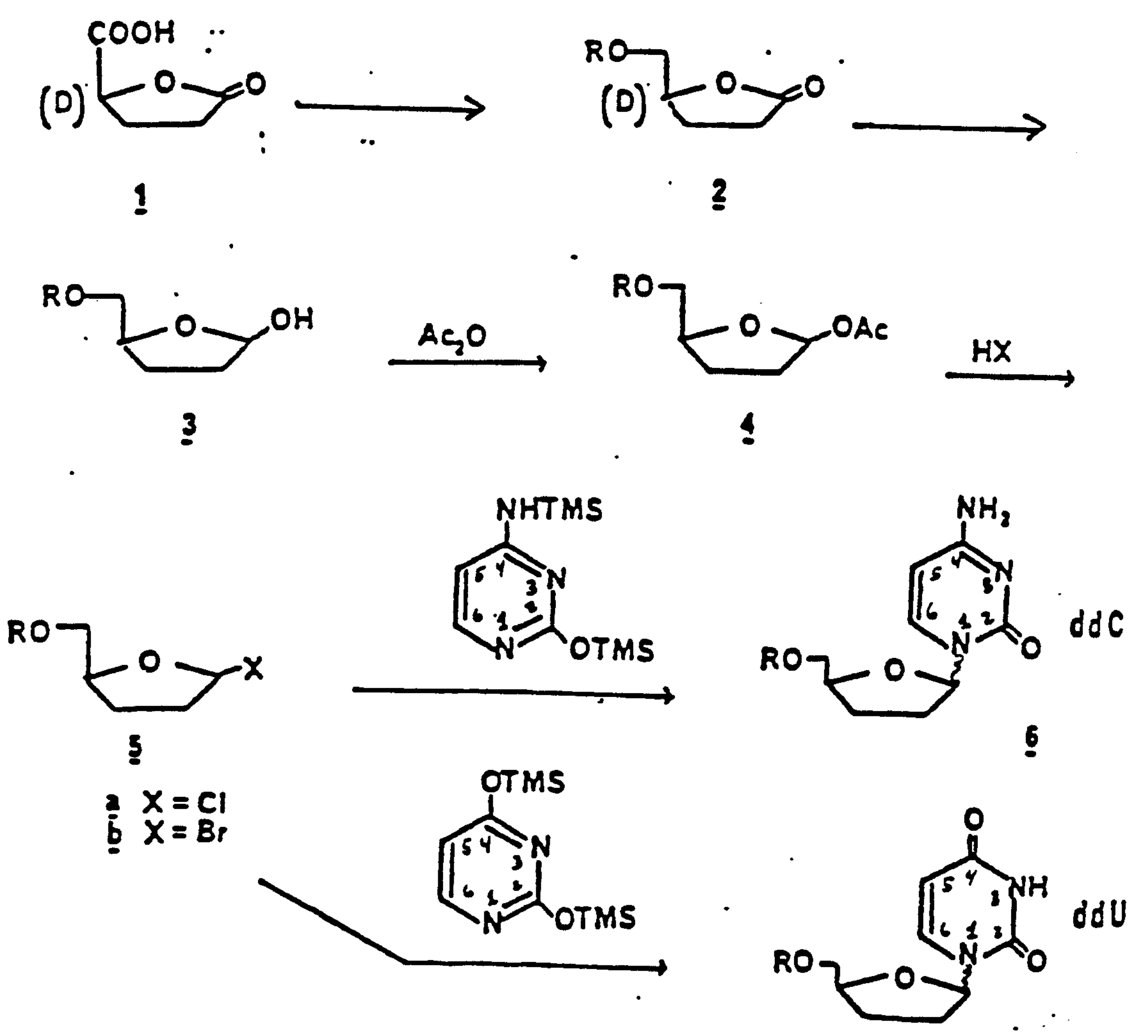


The starting material, γ-carboxy-γ-butyrolactone may be conveniently obtained from L-or D-glutamic acid by utilizing conventional techniques reported in the chemical literature[16].

A combination of chemical reactions produce a suitably blocked, 2,3-dideoxypentofuranose, 3. This compound and its derivatives are known in the literature.

Thus, the conversion of the starting material, γ-carboxy-γ-butyrolactone, to a 5-O-hydroxy-protecting group-methyl-γ-butyrolactone, step (a), involves the reduction of the γ-carboxy group to a hydroxymethyl group followed by reaction with a hydroxy-protecting group reagent. For example, the reduction of the γ-carboxy group and protection of the resulting γ-hydroxymethyl group by means of the benzyl group ($PhCH_2$-), step (a), was achieved by the successive reaction of the starting compound of Formula 1 with $BH_3.SMe_2$ and $PhCH_2Br$[16,17].

The primary alcohol functional group may be protected as an ether, such as a trialkyl or dialkyl aryl or diaryl alkyl or triaryl silyl, unsubstituted and substituted benzyl, unsubstituted and substituted alkyl, or allyl ether, or as an ester, such as a benzoyl, mesitoyl, pivaloyl, unsubstituted or substituted acetyl, or a carbonate ester. See "Protective Groups in Organic Synthesis," T. W. Greene, John Wiley, New York 1981 for more detailed description of protecting groups and the chemistry relating to the same. In a more preferred embodiment, there was used as the hydroxy-protecting group for the primary alcohol group at the 5-position the benzyl group, and most preferably the benzoyl group, because of their stability and well-known preparation.

The conversion of the 5-O-hydroxy-protecting group-γ-butyrolactone, to the 5-O-hydroxy-protecting group-2,3-dideoxypentofuranose having Formula 3 in step (b) was achieved by reacting the intermediate having Formula 2 with NaH and $HCO_2Et$ followed by HCl[5].

It will be understood by those skilled in the art to which this invention relates that any one of a number

of reducing agents may be used to carry out either or both of steps (a) and (b) independently (i.e., in succession) or at the same time. Other useful reducing agents besides $BH_3.SMe_2$ used in steps (a) and (b) as described above include $NaBh_4$, $NaBH_4$ plus LiCl or $AlCl_3$ or $BF_3$, $LiAlH_4$, $LiAlH(OMe)_3$, $LiAlH(O$-t-$Bu)_3$, $(Sia)_2BH$ (disiamyl borane) and other dialkylboranes, and the like. We prefer to use $(Sia)_2BH$ as the reducing agent because of its convenient handling and reactivity.

The step (c) conversion of the intermediate having Formula 3 to the intermediate having Formula 4 bearing an "activated" hydroxy group at the C(1) position of the 2,3-dideoxypentofuranose ring system may be achieved by any reagent effective to convert this hydroxy group to a group which may be displaced readily by Cl or Br upon reaction with HCl or HBr or, preferably, TMSBr or TMSCl (+) catalytic amount of TMSI. ("TMS" represents the tetramethylsilyl group). Such a group which be so displaced readily include O-activating groups selected from alkylcarbonyl, arylcarbonyl, alkylthiocarbonyl, arylthiocarbonyl, alkylsulfonyl, arylsulfonyl and carbonate groups wherein the alkyl moiety may be an unsubstituted or substituted $C_1$-$C_3$ alkyl group and the aryl moiety may be an unsubstituted or substituted phenyl group and wherein the substituent on the alkyl and aryl moieties may be selected from 1 to 3 groups selected from halo and $C_1$-$C_3$ alkoxy groups. More preferably, this activating group is selected from corresponding (to the above) acetoxy and benzoyloxy groups and, most preferably, acetoxy. Step (c) was conveniently carried-out using acetic anhydride/pyridine reagent.

The step (d) displacement of the 1-O-activating group functional group with a leaving group selected from Cl and Br was achieved by treatment of 4 with HCl (or HBr) in $CH_2Cl_2$ at low temperature to give the furanosyl halides 5a and 5b, which are present in solution as a mixture of anomers ($\alpha$ and $\beta$).

In step (e) of the process according to this invention, the intermediate having Formula 5 from step (d) above was reacted with a suitably activated base, wherein the base is selected from purine and pyrimidine bases and wherein such base has been activated by reaction with well-known activating reagents to obtain a silylated, acetylated or benzoylated or other acylated base, in the presence of a suitable polar or non-polar solvent and, optionally, in the presence of a Lewis acid such as, for example, boron halides, aluminum halides, titanium halides, tin (IV) chloride, zinc halides, trimethylsilyl bromide, iodide, triflate and any other species well-known to be used in glycosylation reactions or in the presence of a Bronsted acid such as hydrogen chloride, bromide or iodide. Especially useful in this step (e) is the use of silylated purines and pyrimidines in the presence of non-polar solvents such as, for example, benzene, toluene, chloroform, dichloromethane, dichloroethane and carbon tetrachloride. Examples of useful polar solvents include others such as tetrahydrofuran and dioxane, nitriles such as acetonitrile, dimethylformamide, and dimethylsulfoxide. An example of a useful procedure to carry out step (e) is disclosed in Brundidge et al., U.S. Patent 4,625,020 which discloses the coupling of silylated pyrimidines, wherein active hydrogens of hydroxy and amino groups are blocked by silyl groups such as the trimethylsilyl group, with a 2-deoxy-2-fluoroarabinofuranosyl halide. As this referenced procedure was applied to step (e) according to the present invention, a purine or pyrimidine base having all active hyroxy and amino hydrogens blocked by the trimethylsilyl group was reacted with an intermediate having Formula 5.

Thus, treatment of intermediate having Formula 5 with a silylated base as defined above in $CH_2Cl_2$ and $CHCl_3$ gave protected 2',3-dideoxynucleosides as a mixture of anomers ($\beta$:$\alpha$ = 1:1 to 3:1) in good yields (from about 49% to about 74%).

Alternatively, in another embodiment according to this invention, when in step (c) the group "A" is acetyl, the intermediate from step (c) may be reacted with an activated base in the presence of a Lewis acid as in step (e) so as to obtain the dideoxynucleoside product without proceeding through step (d).

Generally, in order to obtain final products having antiviral, antimetabolic, antineoplastic, and anti-human immunodeficiency viruses (anti-HIV) activity, following the coupling reaction of step (e) and prior to recovery of product in step (f), the process according to the present invention includes the additional step of removing the 5-O-protecting group. Suitable procedures to remove this protecting group are well-known in the field to which this invention relates and examples are disclosed in "Protective Groups in Organic Synthesis" by T. W. Greene, John Wiley, New York, 1981 mentioned above. When the protecting group is the especially preferred benzyl or benzoyl group, it can be easily removed by catalytic hydrogenation ($H_2$ $PdCl_2$) or treatment with ammonia in methanol, ($NH_3$/MeOH), respectively.

The 2',3'-dideoxynucleoside product, unprotected or protected, was conveniently recovered by collecting the reaction mixture from step (e) or from the additional step wherein the 5-O-protecting group was removed, removing insoluble reactants and additives by filtration through Celite, and purifying the resulting product by column chromatography, for example on a silica gel column using a mixture of about 1-5% methanol in chloroform as eluent.

The resulting anomers ($\beta$ and $\alpha$) were separated using chromatographic and crystallization techniques well-known in the field to which this invention relates. The desired active $\beta$-anomer generally was obtained

in greater proportions than the apparently biologically inactive, or at least less biologically active, α-anomer.

Thus, the process according to the present invention affords a stereospecific synthesis for producing 2',3'-dideoxynucleosides including (D)-β-, (D)-α, (L)-β-, and (L)-α-compounds.

The following is a general procedure for separating β and α anomers: the mixture of anomers is applied to a silica gel HPLC column and eluted with a gradient mixture of ethyl acetate and hexane at high pressure (flow rate of 100-500 ml/min). Appropriate fractions are pooled and the solvent removed, e.g. by evaporation under reduced pressure, to afford the pure anomers.

As is mentioned above, the base component, B, of the 2',3'-dideoxynucleoside produced according to the process of the present invention is selected from purine and pyrimidine bases. When derived from purine bases, representative of B are the following:

6-amino-purin-9-yl (adenin-9-yl)
2-aminopurin-9-yl
2,6-diaminopurin-9-yl
2-amino-6-hydroxypurin-9-yl (guanin-9-yl)
6-hydroxypurin-9-yl

In addition to the above, the B component may be 2-halapurin-9-yl, 6-halopurin-9-yl, or 2,6-dihalopurin-9-yl, in which event the base component need not be activated, for example, completely silylated, in order to undergo the condensation or coupling reaction in step (e). When derived from pyrimidine bases, representative of B are the following:

2,4-dihydroxypyrimidin-1-yl
5-methyl-2,4-dihydroxypyrimidin-1-yl
5-ethyl-2,4-dihydroxypyrimidin-1-yl
2-hydroxy-4-aminopyrimidin-1-yl
5-vinyl-2,4-dihydroxypyrimidin-1-yl
5-halovinyl-2,4-dihydroxypyrimidin-1-yl
5-halomethyl-2,4-dihydroxypyrimidin-1-yl
5-haloethyl-2,4-dihydroxypyrimidin-1-yl

The above-mentioned 5-methyl and 5-ethyl substituents are representative of 5-alkyl substituents and the 5-vinyl substituent is representative of 5-alkenyl substituents. Examples of halo-groups on the 5-halovinyl (or 5-haloalkenyl) group include 1 to 3 or even 4 F, Cl, and Br groups.

Thus, the process according to this invention is useful for the preparation of a variety 2',3'-dideoxynucleosides, both pyrimidine and purine nucleosides, having antiviral, antimetabolic, and antineoplastic activity as well as activity against human immunodeficiency viruses.

The compounds according to this invention may be provided as pharmaceutically acceptable salts provided that the salts are compatible with the standard and conventional pharmaceutical carriers and other conventional adjuvants and excipients customarily employed in producing pharmaceutical compositions adapted for oral or parenteral administration. The acid addition salts are formed by conventional techniques involving reaction of the compounds of the invention with a mineral acid or organic carboxylic and sulphonic acids.

Examples of suitable acids include hydrochloric acid, phosphoric acid, acetic acid, citric acid, maleic acid, succinic acid, benzoic acid, tartaric acid, ascorbic acid, methane sulfonic acid, p-toluene sulphonic acid and the like.

The compounds of this invention can be formulated into pharmaceutical compositions. Such compositions comprise one or more of the compounds of the invention in association with a pharmaceutically acceptable carrier. The reference Remingtons Pharmaceutical Sciences, 15th edition by E.W. Martin (Mark Publishing Company , 1975) discloses typical carriers and methods of preparation .

The pharmaceutical carrier may be solid or liquid to provide solid or liquid compositions for topical or systemical administration. By systemic administration is intended oral, rectal, and parenteral (i.e. intramuscular, intervenous, subcutaneous and nasal) routes.

Solid form compositions suitable for oral administration include powders, tablets, capsules, granules and the like. Suitable solid carriers include for example magnesium carbonate and stearate, talc, sugar, lactose, pectine, dextrine, starch,gelatine, cellulosic materials and the like.

The compounds according to the invention may be provided as soluble compounds or compositions, including solutions, suspensions and emulsions which can be dissolved in sterile water or other liquid medium for oral administration or for parenteral administration. Examples of liquid carriers suitable for oral administration include water, alcohol, polypropylene, glycol, polyethylene glycol and mixtures of two or more of the above. Examples of liquid carriers suitable for parenteral use include water-for-injection, physiological saline and other suitable sterile injection media.

In therapeutic use compounds of this invention will be administered in an effective amount. It is to be understood that the actual preferred dosage of compound will vary widely depending upon the requirements of the host being treated, the situs and disease being treated, the composition being and the route of administration. Optimal dosage and administration rates for a given set of conditions may be readily ascertained by those skilled in the art using conventional dosage determination tests.

The following examples illustrate but a few representative embodiments of the process according to this invention and are not to be construed as limiting in scope. All parts and percentages are by weight and temperatures are in degrees Celsius unless otherwise specified.

The following Scheme II is in reference to the following examples 1-9.

SCHEME II

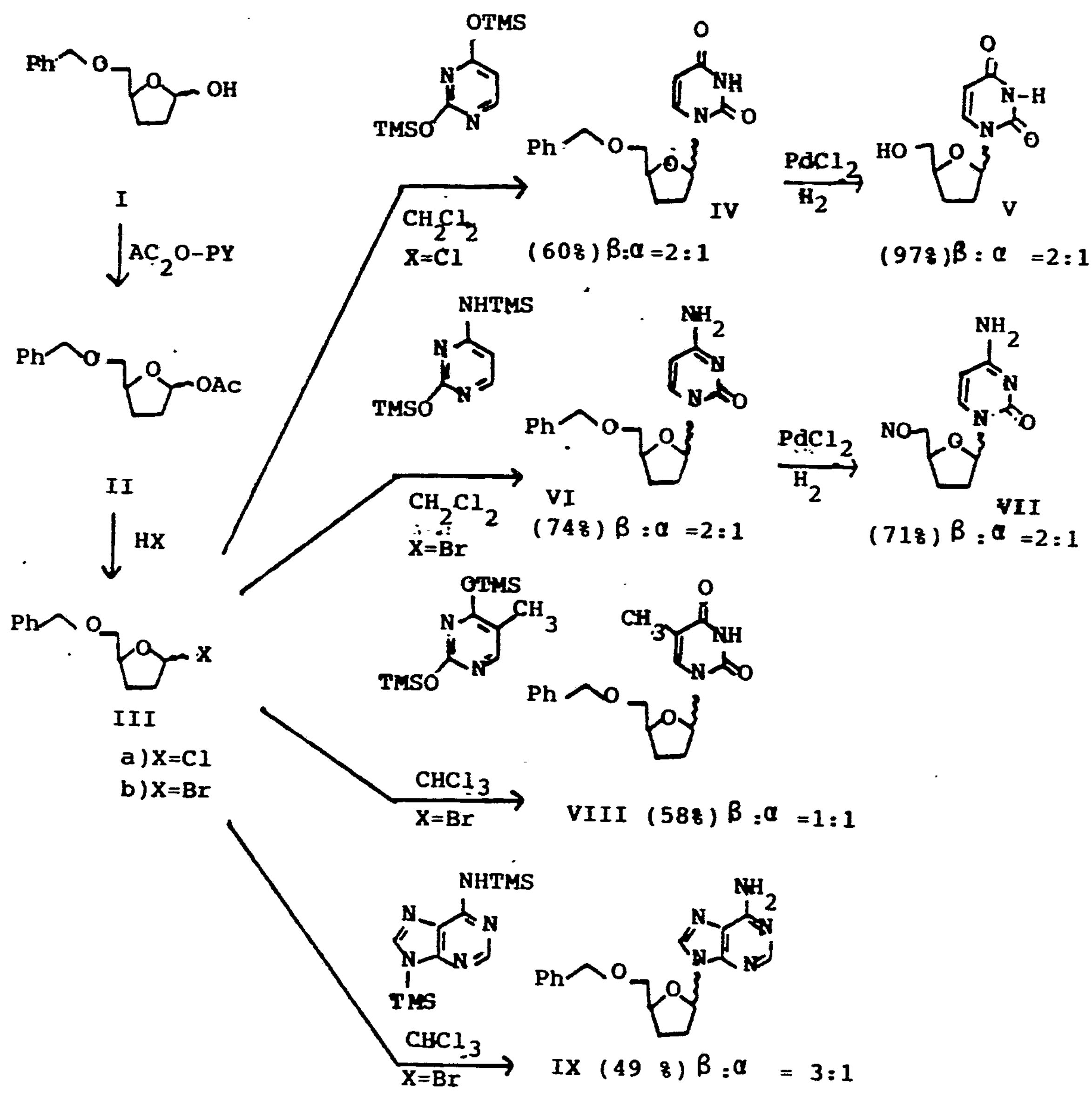


## EXPERIMENTAL

Example 1:

1-O-Acetyl-5-O-Benzyl-2,3-dideoxy-D-pentofuranose (II).

A solution of 1.00 g (0.0048 mole) of 5-O-Benzyl-2,3-dideoxy-D-pentofuranose, I, prepared according to the literature[15,18], in 2 mL dry pyridine and 1 mL acetic anhydride was stirred at room temperature for 2.5 hrs. The reaction mixture was poured onto ice, stirred, and extracted with dichloromethane. The organic layer was washed with 1N hydrochloric acid, saturated aqueous sodium bicarbonate, and brine. The dichloromethane solution was dried over sodium sulfate, filtered and evaporated to dryness, yielding 1.20 g (100%) of 1-O-acetyl-5-O-benzyl-2,3-dideoxy-D-pentofuranose as a colorless liquid. Chromatography on silica gel with a mixture of ethyl acetate and hexane afforded pure II. The nuclear magnetic resonance spectrum was consistent for the desired structure and indicated that II is a 3:2 mixture of anomers.

Crude II is, however, essentially pure and was normally used as such in the next step.

Example 2:

5-O-Benzyl-2,3-dideoxy-D-pentofuranosyl chloride (IIIa).

To a solution of 0.128 g (0.00051 mole) of 1-O-acetyl-5-O-benzyl-2,3-dideoxy-D-pentofuranose in 1 mL dichloromethane at 0°C, 4 mL of a 0.125 N solution of hydrogen chloride (0.00051 mole) in dichloromethane were added. After 1 hr at 0°C, a nuclear magnetic resonance spectrum of a portion of this solution indicated complete conversion to the moisture-sensitive 5-O-benzyl-2,3-dideoxy-D-pentofuranosyl chloride (IIIa). The above solution was used directly in the coupling experiments.

Example 3:

5-O-Benzyl-2,3-dideoxy-D-pentofuranosyl bromide (IIIb).

To a solution of 0.0965 g (0.000384 mole) of 1-O-acetyl-5-O-benzyl-2,3-dideoxy-D-pentofuranose in 1 mL dichloromethane at -30°C, 1 mL of a 0.36 N solution of hydrogen bromide (0.00036 mole) in dichloromethane was added. After 15 min, a nuclear magnetic resonance spectrum of a portion of this solution indicated complete conversion to the moisture-sensitive 5-O-benzyl-2,3-dideoxy-D-pentofuranosyl bromide (IIIb). The above solution was used directly in the coupling experiments.

Example 4:

5'-O-Benzyl-2',3'-dideoxyuridine (IV).

A suspension of 0.24 g (0.00214 mole) uracil, 0.52 mL (0.00247 mole) hexamethyldisilazane and 0.025 g (0.00019 mole) ammonium sulfate in 8 mL dry acetonitrile was refluxed under Argon for 2 hrs. The mixture was cooled, evaporated to dryness, replacing the solvent with 5 mL dry dichloromethane, and yielding a 0.428 M solution of O, O-bis-trimethylsilyluracil. To a solution of 0.104 g (0.000384 mole) of 5-O-benzyl-2,3-dideoxy-D-pentofuranosyl bromide in 2 mL dichloromethane at -40°C, 2 mL of a 0.428 M solution of bis-trimethylsilyl uracil (0.000856 mole) in dichloromethane were added and the temperature was allowed to rise slowly to 25°C. After an overnight period at room temperature, the reaction was quenched with 0.2 mL methanol and filtered through Celite, washing with 30:1 dichloromethane-methanol.

The crude product was chromatographed on silica gel, using 75% ethyl acetate in hexane, to yield 0.105 g (90%) of a mixture of 5'-O-benzyl-2',3'-dideoxyuridine and its C(1) epimer (α-anomer) ($\beta$:$\alpha$ = 2:1).

The nuclear magnetic resonance spectrum was consistent with the structure.

The $\beta$ and $\alpha$ anomers are separable by chromatography on silica gel[20,21].

Example 5:

2',3'-Dideoxyuridine (V).

A suspension of 0.425 g (0.00024 mole) pre-reduced palladium chloride and 0.080 g (0.000265 mole) of 5'-O-benzyl-2', 3'-dideoxyuridine (α and β anomers) in 1.5 mL dry methanol was hydrogenated in a Parr shaker with 15 p.s.i. of hydrogen for 1 hr. The catalyst was removed by filtration through Celite, the filtrate was neutralized with 0.70 g of a Dowex WGR resin for 15 min. at room temperature, filtered again and evaporated. The crude product was purified by silica gel chromatography, eluting with 5% methanol in dichloromethane, yielding 0.0545 g (97%) of 2',3'dideoxyuridine, together with the α anomer (β:α = 2:1).

The nuclear magnetic resonance spectrum of the major component of the mixture was in agreement with literature data [6,15].

Example 6:

5'-O-Benzyl-2',3'-dideoxycytidine (VI).

A suspension of 0.363 g (0.00327 mole) cytosine, 0.78 mL (0.0037 mole) hexamethyldisilazane and 0.038 g (0.00029 mole) ammonium sulfate in 10 mL dry acetonitrile was refluxed under Argon for 2 hrs. The mixture was cooled, the solvent evaporated, replaced with 5 mL dichloromethane and allowed to settle, to yield a 0.654 M solution of O,N-bis-trimethylsilyl cytosine.

To a solution of 0.92 g (0.0004 mole) of 5-O-benzyl-2, 3-dideoxy-D-pentofuranosyl chloride in 4 mL dichloromethane at 0°C, 1.26 mL of a 0.654 M solution of bis-trimethylsilyl cytosine (0.00082 mole) were added and the temperature was allowed to rise slowly to 25°C. After an overnight period at room temperature, the reaction was quenched with 0.2 mL methanol and filtered through Celite, washing with 20:1 dichloromethane-methanol. The crude product was chromatographed on silica gel, eluting with 5% methanol in methylene chloride, to yield 0.090 g (74%) of a mixture of 5'-O-benzyl-2',3'-dideoxycytidine and its C(1) epimer (α anomer) (β:α = 2:1).

The nuclear magnetic resonance spectrum was consistent with the assigned structure. The β and α anomers were resolved on a silica gel plate, although separation of the anomers is not yet completed[20,21].

Example 7:

2',3'-Dideoxycytidine (VII).

A suspension of 0.070 g (0.000395 mole) pre-reduced palladium chloride and 0.040 g (0.000132 mole) 5'-O-benzyl-2',3'-dideoxycytidine (α and β anomers) in 1.5 mL dry methanol was hydrogenated in a Parr shaker with 15 p.s.i. of hydrogen for 1 hr.

The catalyst was removed by filtration through Celite, the filtrate was neutralized with 0.1 g of an Amberlite IRA resin for 15 min at room temperature, filtered again and evaporated. The crude product was purified by silica gel chromatography, eluting with 25% methanol in dichloromethane, yielding 0.020 g (71%) of 2',3'-dideoxycytidine, together with the α anomer (β:α = 2:1).

The nuclear magnetic resonance spectrum of the major component of the mixture was in agreement with literature data[5].

Example 8:

5'-O-Benzyl-2' ,-3'-deoxythymidine (VIII).

A suspension of 0.252 g (0.002 mole) thymine, 0.5 mL (0.0024 mole) hexamethyl-disilazane and 0.01 g (0.0001 mole) iodotrimethylsilane was refluxed for 48 hrs., then cooled. This solution of O,O-bis-trimethylsilyl thymine was added to a solution of 0.271 g (0.001 mole) 5-O-benzyl-2,3-dideoxy-D-pentofuranosyl bromide in 12.1 mL dry chloroform at -78°C.

The mixture was slowly allowed to reach room temperature and stirred overnight. The reaction was quenched with 0.5 mL methanol, diluted with dichloromethane, washed with water and brine and dried over sodium sulfate.

The crude product was chromatographed on silica gel, eluting with 4:1 ethyl acetate hexane. The yield

of 5'-O-benzyl-3'-deoxythymidine (both anomers obtained, $\alpha:\beta$ = 1:1) was 0.180 g (58%).

The nuclear magnetic resonance spectrum was consistent with the structure. Although separation of the anomers was not carried out, chromatographic methods are successfully employed for these separations in nucleoside chemistry[20,21].

Example 9:

5'-O-Benzyl-2',3'-dideoxyadenosine (IX).

A suspension of 0.270 g (0.002 mole) adenine, 0.5 mL (0.0024 mole) hexamethyldisilazane and 0.01 g (0.001 mole) iodotrimethylsilane was refluxed for 18 hrs. then cooled.

This solution of N,N-bis-trimethylsilyl adenine was added to a solution of 0.271 g (0.001 mole) 5-O-benzyl-2,3-dideoxy-D-pentofuranosyl bromide in 12.1 mL dry chloroform at -78°C. The mixture was slowly allowed to reach room temperature and stirred overnight. The reaction was quenched with 0.5 mL methanol, diluted with dichloromethane, washed with water and brine and dried over sodium sulfate. The crude product was chromatographed on silica gel, eluting with 8% methanol in dichloromethane. The yield of 5'-O-benzyl-2',3'-dideoxyadenosine ($\beta:\alpha$ ratio = 3:1) was 0.158 g (49%).

The nuclear magnetic resonance spectrum was consistent with the structure. Although separation of the anomers shown in SCHEME II is not yet completed, this may be achieved by the chromatographic methods typically used in nucleoside chemistry[20,21]

The following SCHEME III is in reference to the following examples 10-14 and 16 and 17. The biological activity of representatives of the novel compounds is set forth in the accompanying Table - Biological Activity Against Human Immunodeficiency Virus (HIV).

SCHEME III

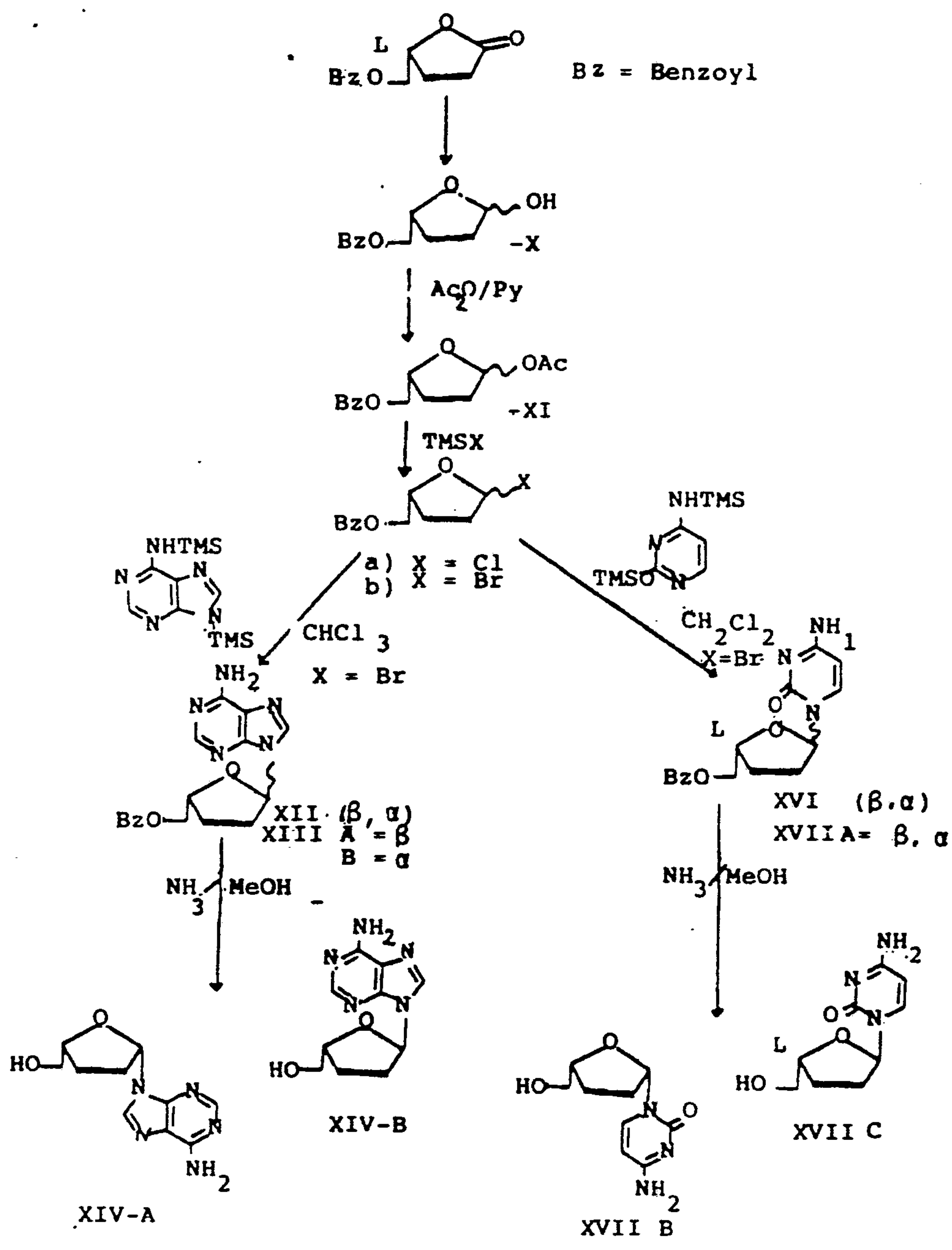


Example 10:

(L)-5-O-Benzoyl-2,3-dideoxypentofuranose, X.

To 200 mL of a 0.5 M solution of disiamylborane in THF solution at 0°C was added dropwise under nitrogen a solution of 15.0g (0.068 mole) of (L)-5'-benzoyloxy-5-hydroxymethylbutyrolactone in 40 mL of dry tetrahydrofuran. After 20 min at 0°C the reaction was warmed to 22°C. The reaction was stirred at 22°C for 1 1 2 hrs, then worked up by slowly adding 12 mL of water and refluxing for 30 min. The reaction mixture was cooled to 0°C followed by the slow addition of 24 mL of 30% hydrogen peroxide maintaining the pH

between 7-8 with 1N sodium hydroxide. After the addition the reaction was evaporated under reduced pressure on a rotovapor at 30°C to an oily residue. This was partitioned between 500 mL dichloromethane and 150 mL water. The aqueous layer was extracted with 2 x 100 mL dichloromethane and then the combined organic layers were washed with 50 mL water, dried over anhydrous sodium sulfate and evaporated to an oil. Yield = 15.0g (99%) of (L) 5-O-benzoyl-2,3-dideoxypentofuranose. The $^1$H-NMR was consistent for structure and the oil was used directly in the next step.

Example 11:

1-O-Acetyl-(L)-5-O-benzoyl-2,3-dideoxypentofuranose. XI.

A solution of (L)-5-O-benzoyl-2,3-dideoxypentofuranose (12.0g 0.054 mole) in 24 mL of pyridine and 12 mL of acetic anhydride was stirred at 22°C for 2 1/2 hrs then worked up by adding 100 g of ice then 600 mL of ethylacetate. The reaction mixture was then washed with 3 X 100 mL 1N hydrochloric acid 4 X 100 mL saturated aqueous sodium bicarbonate and 100 mL of brine. The organic layer was dried over sodium sulfate and evaporated to yield a pale yellow oil. This could be used as is or chromatographed on silica gel 35% --->50% EtOAc/hexane. Yield: 8.6g (60%) of 1-O-acetyl-(L)-5-O-benzoyl-2,3-dideoxy-pentofuranose. Its NMR was consistent for structure. Other fractions contained the desired material but were impure.

Example 12:

α,β-(L)-5′-o-benzoyl-2′,3′-dideoxyadenosine. XII.

1-O-acetyl-(L)-5-O-benzoyl-2,3-pentofuranose (2.97g, 0.01125 mole) was dissolved in 20 mL of 1,2-dichloroethane. To this was added 1.44 mL of trimethylsilylbromide. This was stirred at 22°C for 20 min prior to the addition of 28.93 mL of a 0.428 M solution of bis-silyladenine in 1,2-dichloroethane. The solution was stirred for 120 hrs at 22°C. The reaction was then worked up by cooling to 0°C, followed by pouring into 50 mL of cold saturated aqueous sodium bicarbonate. The reaction mixture was partitioned between 300 mL dichloromethane and 2 X 50 mL of water. The organic layer was dried and evaporated to yield a colorless oil 3.3g (87%) of a mixture of products.

Example 13:

Separation of α and β anomers of 9-(L)-5′-O-benzoyl-2′ ,3′-dideoxyadenosine (XIII, A and B).

This material was separated using a Waters LC-500 chromatography system on a Waters C-18 prep pak cartridge equilibrated and eluted with 60% MeOH/$H_2O$ as solvent. The sample was dissolved in 4 mL of methanol and injected. Elution was carried out at 0.1 L/min using a refractive index detector. Fractions were collected and analyzed by HPLC. Similar ones were pooled, evaporated to 1/3 volume and partitioned between 3 X 500 mL of dichloromethane. The pooled organic layers were dried and evaporated to yield pure 9-(β)-(L)-5′-O-benzoyl-2′,3′-dideoxyadenosine) 660mg [NMR consistent with structure] and pure 9-(α)-(L)-5′-O-benzoyl-2′,3′-dideoxyadenosine 560 mg [NMR consistent with structure.]
There was also obtained a mixture of the 9-(α,β)-(L)-5′-O-benzoyl-2′,3′-dideoxyadenosine) (≈1.8 g).

Example 14:

9-(β)-(L)-2′,3′-dideoxyadenosine (XIV A).

9-(β)-(L)-5′O-benzoyl-2′,3′-dideoxyadenosine) (270 mg, .0008 mole) was dissolved in 5 mL of methanol. To this was added 20 mL of methanol saturated with ammonia. This was stoppered and vented to a bubbler. After 16 hr the reaction was complete and then was evaporated. The residue was chromatographed on a silica gel column packed and eluted with 10% methanol in dichloromethane. A gradient was run to 20% methanol in dichloromethane. Desired pure fractions were pooled and evaporated. The residue was

crystallized from methanol to yield 160 mg (85%) of 9-(β)-(L)-2',3'-dideoxyadenosine). [NMR was consistent for structure.]
Calculated for C 51.05, H 5.57. N 29.77
Found C 51.15. H 5.63, N 29.67

9-(α)-(L)-2',3'-dideoxyadenosine (XIV B).

9-(α)-(L)-5'-O-Benzoyl-2',3'-dideoxyadenosine) (250 mg, .00074 mole) was dissolved in 5 mL of methanol. To this was added 20 mL of methanol saturated with ammonia. This was stoppered and vented to a bubbler. After 16 hours the reaction was evaporated and chromatographed on a silica gel column packed and eluted with 10% methanol in dichloromethane. A gradient was run to 20% methanol in dichloromethane. Desired pure fractions were pooled and evaporated then crystallized from methanol to yield 170 mg (98%) of 9-(α)-(L)-2',3'-dideoxyadenosine). [$^1$H NMR consistent with structure.]
Calculated for C 51.05, H 5.57, N 29.77
Found C 51.04, H 5.59, N 29.64

Example 15:

Separation of α,β-(D)-5'-O-benzoyl-2',3'-dideoxyadenosine (XV A).

The mixture from the coupling reaction containing 9-(α,β)-(D)-5'-O-benzoyl-2',3'-dideoxyadenosine was reverse phase chromatographed on a Waters LC-500 chromatography system using a Waters prep pak C-18 column and 50% MeOH/$H_2O$ to elute. This material was dissolved in 2 mL of methanol and injected. Flow rate of .1 L/min was used. After eluting 1.5 L of solvent the 4 L of mobile phase was gradually deluted with 60% MeOH/$H_2O$. The fractions containing mainly the α-(D)-5'-O-benzoyl isomer were then evaporated. The residue was then rechromatographed on a flash column 1" x 14" packed with C-18 and eluted with 50% methanol/water to a gradient of 65% methanol/water.
Fractions were collected and pure ones pooled and evaporated to 1/3 volume. This was then extracted 5 X 100 mL with dichloromethane and the pooled organic layers were dried over anhydrous sodium sulfate and evaporated. This material was crystallized from acetone to yield α-(D)-5'-O-benzoyl-2',3'-dideoxyadenosine. [NMR was consistent for structure.]

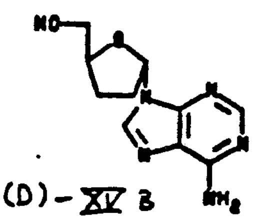

(D)-XV B

9-α-(D)-2',3'-dideoxyadenosine (XV B).

The material 9-(α-(D)-5'-o-benzoyl-2',3'-dideoxyadensoine) 220 mg (0.00065 mole) was dissolved in 8 mL of methanol saturated with ammonia. This was stirred in a closed system for 16 hrs. The reaction was then evaporated and chromatographed on a silica gel column packed and eluted with 12% methanol in dichloromethane. Fractions were collected and pure ones pooled and evaporated.
The residue was crystallized from methanol to yield 9-(α)-(D)-2',3'-dideoxyadenosine) 114 mg 75%. [$^1$H NMR was consistent with structure.]

Example 16:

(α,β)-(L)-5'-O-benzoyl-2',3'-dideoxycytidine (XVI).

1-O-acetyl-5-(L)-O-benzoyl-2,3-pentofuranose 2.815 g (10.66 mm) was dissolved in 20 mL of 1,2-dichloroethane. To this was added 1.8 mL of trimethylsilylbromide (15.46 mm) (1.45 eq.) under Argon at 22°C. this was stirred at 22°C for 20 min followed by cooling to a solid mass at -78°C. To this was then added 50 mL a solution of a .31 molar solution of bissilylcytosine (15.65 mm). The bath was removed and the reaction was allowed to warm to 22°C. After 16 hrs the reaction was worked up by pouring into 50 mL of cold saturated aqueous sodium bicarbonate. This was partitioned with 300 mL dichloromethane ($CH_2Cl_2$) and the organic layer was washed with 50 mL of water. The organic layer was dried over anhydrous sodium sulfate and evaporated to yield 3.36 g of a mixture containing α,β-(L)-5'-O-benzoyl-2',3'-dideoxycytidine.

Example 17:

Separation of α,β-(L)-5'-O-benzoyl-2',3'-dideoxycytidine (XVII A).

The material isolated from the coupling reaction was separated on a Waters LC-500 chromatography system using a reverse phase C-18 chromatography cartridge (Waters C-18 prep pak) equilibrated and eluted with 35% MeOH/65% pH 6.5 .01 M ammonium phosphate buffer. The column was eluted at .1 L min using a gradient over a total of 6 L to 50% MeOH/50% pH 6.5 buffer.

Fractions were collected and analyzed by TLC on $SiO_2$ plates with 5% MeOH/$CH_2Cl_2$ using 2 developments. Fractions containing mainly the first spot on TLC were pooled and evaporated. To further purify, the material was then flash chromatographed on a C-18 column 1" X 14" eluted with 40% MeOH/60% buffer pH 6.5. After pooling the desired pure fractions and evaporating to 1/3 the volume, the material was partitioned between 3 X 500 mL dichloromethane ($CH_2Cl_2$). The combined organic layer were dried and evaporated to yield 380 mg of β-(L)-5'-O-benzoyl-2',3'-dideoxycytidine. [NMR consistent for structure.]

Similarly, the fractions from the LC-500 chromatograph containing mainly the second spot were pooled and evaporated. This material was further purified on a flash C-18 column 1" X 14" packed and eluted with 40% methanol/60% pH 6.5 buffer. After pooling the desired pure fractions they were evaporated to 1/3 volume and the material was partitioned between 3 X 500 mL of dichloromethane. The combined organic layers were dried and evaporated to yield 506 mg of α-(L)-5'-O-benzoyl-2',3'-dideoxycytidine. [$^1$H NMR consistent with structure.]

β-(L)-2',3'-dideoxycytidine (XVII B).

β-(L)-5'-O-benzoyl-2',3'-dideoxycytidine (250 mg, 0.00079 mole) was dissolved in 5 mL of methanol and 20 mL of methanol saturated with ammonia was added. The reaction was vented through a bubbler. The reaction was stirred at 22°C for 16 hrs. The reaction was complete and then evaporated. The residue was crystallized from ethanol/ether to yield β-(L)-2',3'-dideoxycytidine (132 mg, 78%). [$^1$H NMR was consistent for structure.]

α-(L)-2',3'-dideoxycytidine (XVII C).

α-(L)-5'-O-benzoyl-2',3'-dideoxycytidine (250 mg, 0.00079 mole) was dissolved in 5 mL of methanol and to this was added 20 mL of methanol saturated with ammonia. The reaction was vented to a bubbler. After stirring 16 hours at 22°C the reaction was complete. The reaction was then evaporated and triturated with ether to leave an oil. This was crystallized from methanol/ether to yield α-(L)-2',3'-dideoxycytidine (140 mg, 84%). [$^1$H NMR was consistent for structure.]

Example 18:

Separation of α,β-(D)-5'-O-benzoyl-2',3'-dideoxycytidine (XVIII A).

The mixture of α,β-(D)-5'-O-benzoyl-2',3'-dideoxycytidine was flash chromatographed on a reverse phase C-18 column (1" X 14") packed and eluted with 40% MeOH/pH 6.5 .01 M ammonium phosphate buffer. Fractions were collected and analyzed by TLC on silica gel plates using 5% methanol in dichloromethane as solvent using two developments.
Fractions containing mainly the second spot were pooled and evaporated, then rechromatographed on the same column in the same way. This yielded pure fractions which were pooled and evaporated. The residue was dissolved in 5% methanol in dichloromethane and filtered through glass wool (to eliminate buffer salts). The filtrate was evaporated to yield α-(D)-5'-O-benzoyl-2',3'-dideoxycytidine 190 mg. ['H NMR consistent with structure.] Also obtained during this separation in an analogous manner were pure β-D-ddC and the same mixture as originally started with.

α-(D)-2'-,3'-dideoxycytidine (XVIII B)

α-(D)-5'-O-benzoyl-2',3'-dideoxycytidine 80 mg (0.00025 mole) was dissolved in 5 mL of methanol followed by the addition of 10 mL of methanol saturated with ammonia. The reaction was vented through a bubbler. After 16 hours the reaction was complete and was then evaporated. the residue was triturated with ether to yield 50 mg α-(D)-2',3'-dideoxycytidine. ['H NMR consistent with structure.]

Example 19:

9-α-(L)-2',3'-dideoxyinosine

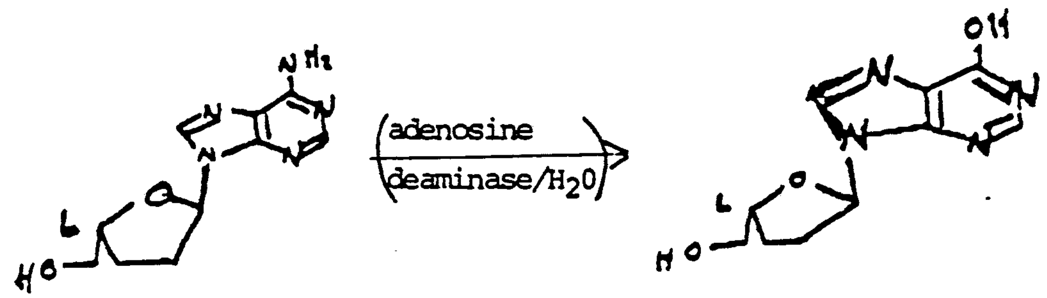


To 45 mg (.00019 Mole) of 9-α-(L)-2',3'-dideoxydenosine was added 3ml of distilled $H_2O$ and 45mg of adenosine deaminase (Sigma, Type II, E (3.5.4.4). The reaction was stirred at 22°C for 18 hours. At this time HPLC analysis (20% MeOH/ph 6.5 ammonium phosphate buffer, c-18 column) indicated the reaction to be 97% complete. The $H_2O$ was then evaporated, followed by combining this reaction with a previous 10mg scale reaction. The was then chromatographed on a silica gel column packed and eluted with 10% $MeOH/CH_2Cl_2$.
Fractions were collected, analyzed and pure ones pooled to yield 42 mg (.00018 Mole) of 9-α-L-2',3'-dideoxyinosine (76% yield) mp >250°C. [NMR was consistent for structure.]

Table - Biological Activity Against Human Immunodeficiency Virus (HIV), μl/ml

| Compound Formula | Compound Name | $ID_{50}$ | $TCID_{50}$ |
|---|---|---|---|
| 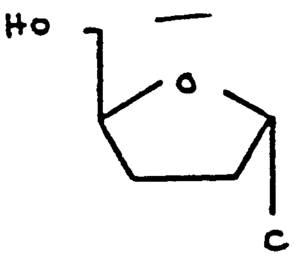 | α-D-ddc (Ex. XVIII B) | <0.1 | <1.0 |
| 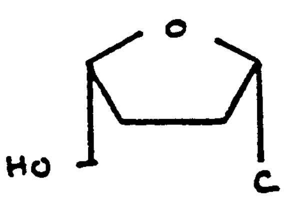 | β-L-ddc (Ex. XVII B) | <0.1 | 22.0 |
| 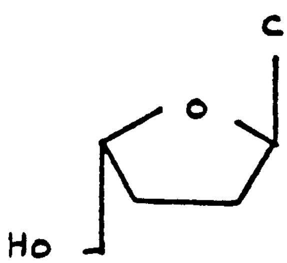 | α-L-ddc (Ex. XVIIC) | 10.0 | >100. |

REFERENCES:


1. Samukov, V.V.; Ofitserov, V.I. Bioorg. Khim. 1983, 9, 132.
2. Prisbe, E.J.; Martin, J.C. synth. Commun. 1985, 15, 401.
3. Horwitz, J.P.; Chua, J.; Noel, M.; Donatti, J.T. J. Org. Chem. 1967, 32, 817.
4. Robbins, M.J. et at.. J. Am. Chem. Soc. 1976, 98, 8213.
5. Marumoto, R.; Honjo, M. Chem. Pharm. Bull. 1974, 22, 128.
6. Furukawa, Y. et al. Chem. Pharm. Bull. 1970, 18, 554.
7. McCarthy, J.R. et al. J. Am. Chem. Soc. 1966, 88, 1549.
8. Robins, M.J. et al. Tet. Lett. 1984, 25, 367.
9. David, S.; Sennyey, G. Carbohydrate Res. 1980, 82, 45.

10. Adachi, T. et al. J. Org. Chem. 1979, 44, 1404.
11. Thiem, J.: Rasch, D. Nucleosides and Nucleotides 1985, 4. 487.
12. Verheyden, J.P.H.; Moffat, J.G. US 3,817,982 (1974).
13. Saito, I. et al. J. Am. Chem. Soc. 1986, 108, 3115.
14. Lin, T.S. et al. J. Med. Chem. in the press.
15. Kaulina, L.T. et al. Khim. Geterosikl. Soedin. 1982, 101.
16. Taniguchi, M.; Koga, K.; Yamada, S. Tetrahedron 1974, 30, 3547.
17. Ravid, U.; Silverstein, R.M.; Smith, L.R. Tetrahedron, 1978, 34, 1449.
18. Hubbard, A.J. et al. Nucleic Acid Res. 1984, 17, 6827.
19. Stone, K.; Little, R.D. J. Am. Chem. Soc. 1985, 107, 2495.
20. Bobek, M.; Martin, V. Tetrahedron Letters 1978, 1919.
21. Woenckhaus, C.; Jeck, R. Liebigs Ann. Chem. 1970, 736, 126.

## Claims

1. A process for producing a 2',3'-dideoxynucleoside represented by the formula

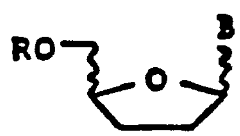

wherein B is a base selected from the group of purine and pyrimidine bases and R is a member selected from H and hydroxy-protecting groups, which comprises the steps of:

(a) converting a member of the group consisting of L-and D-γ-carboxy-γ-butyrolactone of the formula

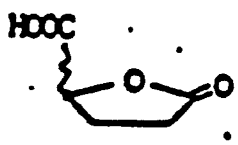

Formula 1

to a 5-O-hydroxy-protecting group-methyl-γ-butyrolactone of the formula

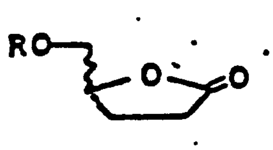

Formula 2

wherein R is a hydroxy-protecting group, by reacting the compound of Formula 1 with a carboxy group reducing agent followed by reacting the resulting hydroxymethyl group with a hydroxy-protecting group reagent;

(b) converting the intermediate from step (a), Formula 2, to the 5-O-hydroxy-protecting group-methyl-2,3-dideoxy-D-or -L-pentofuranose of the formula

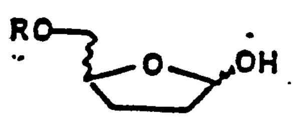

Formula 3

wherein R is a hydroxy-protecting group, by reacting the compound of Formula 2 with a carbonyl group reducing agent;

(c) converting the intermediate from step (b), Formula 3, to a 1-O-activating-group-5-O-hydroxy-protecting group-2,3-dideoxy-D-or -L-pentofuranose of the formula

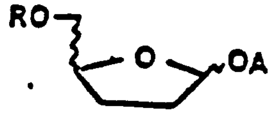

Formula 4

wherein R is a hydroxy-protecting group and A is an O-activating group selected from alkylcarbonyl, arylcarbonyl, alkylthiocarbonyl, arylthiocarbonyl, alkylsulfonyl, arylsulfonyl and carbonate groups, wherein the alkyl moiety may be an unsubstituted or substituted $C_1$-$C_3$ alkyl group and the aryl moiety may be an unsubstituted or substituted phenyl group and wherein the substituent on the alkyl and aryl moieties may be selected from 1 to 3 groups selected from halo and $C_1$-$C_3$ alkoxy groups, by reacting the compound of Formula 4 with an acylating or sulfonating or carbonylating agent corresponding to the group A above;

(d) converting the intermediate from step (c), Formula 4, by reaction with a compound having one of the formulas HX and TMSX to the 1-leaving-group-5-O-protecting group-2,3-dideoxy-D-or -L-pentofuranose of the formula

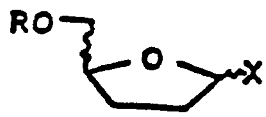

Formula 5

wherein R is a hydroxy-protecting group and X is a leaving group selected from F, Cl, Br and I by reacting the compound of Formula 4 with a compound having one of the formulas HX and TMSX;

(e) reacting the intermediate from step (d), Formula 5 with an activated base selected from purine and pyrimidine bases, wherein the base has been activated by means of reacting the pendant amino and hydroxy groups on the nucleus of the given base with an activating compound selected from silylating and acetylating and benzoylating agents, in the presence of one of a Bronsted acid and a Lewis acid and in the presence of a solvent selected from polar and non-polar solvents; and

(f) recovering the dideoxynucleoside from step (e) above.

2. A process according to claim 1 wherein the reducing agent used in steps (a) and (b) is selected from $BH_3(B_2H_6)$, $BH_3.SMe_2$ $NaBH_4$, $NaBH_4$ plus one of LiCl and $AlCl_3$ and $BF_3$, $LiAlH_4$, $LiAlH(OMe)_3$, and LiAlH-(O-t-BU)$_3$, and dialkylboranes including $(Sia)_2BH$..

3. A process according to claim 1 wherein the reducing agent used in step (a) is $BH_3$ or $BH_3.SMe_2$.

4. A process according to anyone of the preceding claims wherein the hydroxy-protecting group used in step (a) is selected from unsubstituted and substituted benzyl, trialkylsilyl, alkylarylsilyl, unsubstituted and substituted alkyl, vinyl, benzoyl, mesitoyl, pivaloyl, unsubstituted and substituted acetoxy, and carbonate groups.

5. A process according to claim 4 wherein the hydroxy-protecting group is benzyl.

6. A process according to anyone of the preceding claims wherein the O-activating group used in step (c) is selected from alkylcarbonyl and arylcarbonyl.

7. A process according to claim 6 wherein the O-activating group used in step (c) is selected from acetyl and benzoyl.

8. A process according to anyone of the preceding claims wherein in step (e) the base, B, component is selected from purine and pyrimidine bases wherein (1) the bases are activated by reaction with a halotrialkylsilane as the silylating agent wherein the halo is selected from bromo and iodo and (2) the bases are selected from halopurine bases which are sufficiently reactive with the1-leaving-group-5-O-protecting group-2′,3′-dideoxypentofuranose having Formula 5 so as not to require activation of the base by reaction with an activating compound.

9. A process according to claim 8 wherein the base component, B, is selected from purine and pyrimidine bases that are activated by reaction with a halotrimethylsilane and wherein the reaction in step (e) is carried out in a non-polar selected from $CHCl_3$, $CHCl_2$, $ClCH_2$-$CH_2Cl$, and $CCl_4$

10. A process according to claim 9 wherein the base, B, component is selected from the group of purine bases consisting of 6-aminopurin-9-yl, 2-aminopurin-9-yl, 2,6-diaminopurin-9-yl, 2-amino-6-hydroxypurin-9-yl and 6-hydroxypurin-9-yl and the group of pyrimidine bases consisting of 2,6-dihydroxypyrimidin-3-yl, 5-methyl-2,6-dihydroxypryimidin-3-yl, 2-hydroxy-6-aminopyrimidin-3-yl, 5-vinyl-2,6-dihydroxypyrimdin-3-yl, 5-halovinyl-2,6-dihydroxypyrimidin-3-yl, 5-halomethyl-2,6-dihydroxypyrimidin-3-yl, and 5-haloethyl-2,6-dihydroxypryrimidin-3-yl.

11. A process according to anyone of the preceding claims including the additional step, following step (e) and before step (f), of removing the 5-O-hydroxy-protecting group.

12. A process according to claim 11 wherein the 5-O-hydroxy protecting group is selected from benzyl and benzoyl groups and said group is removed by means selected from catalytic hydrogenation of the benzyl group using $H_2$/$PdCl_2$ reagent and $NH_3$ hydrolysis of the benzoyl group, respectively.

13. A compound having the formula

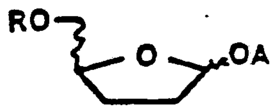

wherein R is a hydroxy-protecting group and A is an O-activating group selected from alkylcarbonyl, arylcarbonyl, alkylthiocarbonyl, arylthiocarbonyl, alkylsulfonyl, arylsulfonyl and carbonate groups.

14. A compound according to claim 13 wherein R is $PhCH_2$ (benzyl) and wherein A is $CH_3CO$ (acetyl).

15. A compound having the formula

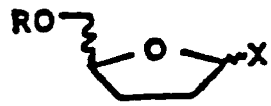

wherein R is a hydroxy-protecting group and X is selected from F, Cl, Br and I.

16. A compound according to claim 15 wherein R is selected from $PhCH_2$ (benzyl) and benzoyl groups.

17. The compounds having the formulae:

D(α)ddA 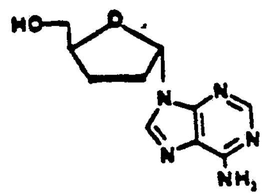

and

D(α)ddC 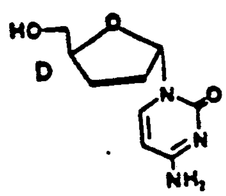

and the pharmaceutically acceptable salts thereof.

18. An (L)-2',3'-dideoxynucleoside having the formula

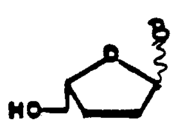 wherein

B is a member selected from the group consisting of purine and pyrimidine bases, and the pharmaceutically acceptable salts thereof.

19. The compounds according to claim 18 having the formulae

L(β)ddA 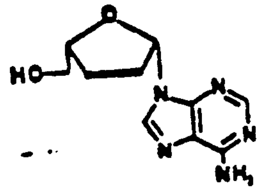 ,

L(α)ddA 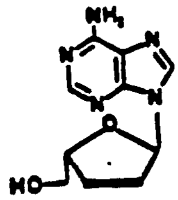 ,

L(β)ddC 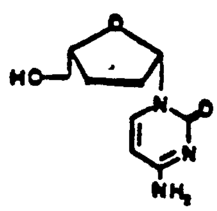

L(α)ddC 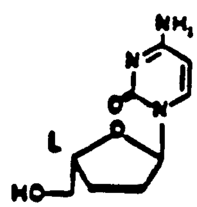

and

L(α)ddI 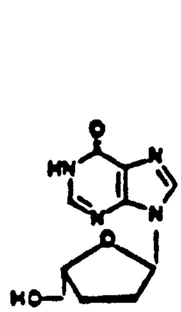

20. A pharmaceutical composition comprising at least one 2',3'-dideoxynucleoside as defined in claims 17 to 19, optionally together with a pharmaceutically acceptable carrier and/or diluent.

21. A process for preparing the pharmaceutical composition of claim 20 which comprises providing a compound as defined in claims 17 to 19 in the form of a pharmaceutical composition and optionally incorporating it into a pharmaceutically acceptable carrier and/or diluent.